# FASCICULE DE BREVET EUROPEEN

(11) **EP 0 751 107 B1**
(45) Date de publication et mention de la délivrance du brevet: **13.11.2002**
(21) Numéro de dépôt: 96401150.6
(22) Date de dépôt: 29.05.1996
(51) Int. Cl.: C07C 17/20, C07C 19/08

(54) **Synthèse du difluorométhane**
Synthese von Difluormethan
Synthesis of difluoromethane

(30) Priorité: 27.06.1995 FR 9507705
(43) Date de publication de la demande: 02.01.1997
(73) Titulaire: Atofina, 92800 Puteaux (FR)
(72) Inventeur: Requieme, Benoît, 69390 Charly (FR); Lacroix, Eric, 69480 Amberieux D'Azergues (FR); Lantz, André, 69390 Vernaison (FR)
(74) Mandataire: Leboulenger, Jean

(56) Documents cités:
- EP-A- 0 328 127
- EP-A- 0 554 165
- US-A- 2 745 886
- DATABASE WPI Section Ch, Week 7636 Derwent Publications Ltd., London, GB; Class E16, AN 76-67559X XP002015039 & JP-A-51 082 206 ( ASAHI GLASS KK) , 20 Juillet 1976

## Description

La présente invention concerne le domaine des hydrocarbures fluorés et a plus particulièrement pour objet la fabrication du difluorométhane (F32) par fluoration du chlorure de méthylène.

Le difluorométhane, connu sous la désignation de F32, est sans danger pour la couche d'ozone. Il est donc particulièrement intéressant pour la substitution des CFC. En mélange avec d'autres hydrofluoroalcanes, tels que le 1,1,1-trifluoroéthane (F143a), le 1,1,1,2-tétrafluoroéthane (F134a) ou le pentafluoroéthane (F125), il est notamment destiné à remplacer le F22 (chlorodifluorométhane) et F502 (mélange azéotropique de F22 et de chloropentafluoroéthane) dans le domaine de la réfrigération, de l'air conditionné et dans d'autres applications.

Il existe différents procédés connus pour la synthèse du F32. L'hydrogénolyse du F12 (dichlorodifluorométhane) ou du F22 (brevets JP 60-01731 et EP 508 660) présente l'inconvénient d'être généralement peu sélective et de sous-produire du méthane non valorisable. Il a récemment été proposé de produire du F32 par fluoration du bis (fluorométhyl) éther (brevet EP 518 506).

Il est également possible de produire du F32 par fluoration du chlorure de méthylène (F30) par de l'HF anhydre. De nombreux brevets décrivent cette réaction, revendiquant l'utilisation de catalyseurs tels que Cr₂O₃, CrF₃, AlF₃, Cr/charbon, Ni/AlF₃.

La difficulté de cette réaction réside dans la stabilité du catalyseur qui a tendance soit à coker rapidement soit à cristalliser. Le problème devient très délicat si l'on veut combiner une forte productivité et une bonne sélectivité tout en maintenant une bonne stabilité du catalyseur.

Pour réduire cette désactivation, il a été proposé l'utilisation de catalyseurs spécifiques tels qu'un mélange mécanique d'alumine et d'oxyde de chrome (brevet GB 821 211). Ce brevet donne un exemple sur la fluoration du chlorure de méthylène mais les productivités en F32 obtenues sur ce catalyseur sont faibles (< 200 g/h/l) et la durée cumulée des tests est inférieure à 5 heures.

Plus généralement, lors des réactions de fluoration, il est très souvent envisagé d'injecter en continu de l'oxygène ou de l'air pour augmenter la durée de vie des catalyseurs. Ainsi le brevet JP 51-82206 revendique l'usage de 0,001 à 1 % d'oxygène pour maintenir l'activité d'un catalyseur contenant principalement de l'oxyde de chrome et éventuellement d'autres oxydes métalliques. Il est indiqué que l'utilisation de plus de 1% d'oxygène provoque l'apparition de réactions secondaires et il est donc préconisé d'utiliser préférentiellement de 0,005 à 0,1% d'oxygène. Dans ce brevet, les réactions de fluoration sont réalisées entre 100 et 500°C et préférentiellement entre 250 et 350°C. De plus, il y est précisé qu'à partir de 200°C, l'activité catalytique est maintenue par l'apport d'oxygène. Bien que ce brevet cite, parmi les réactions, la fluoration de CCl₄, CHCl₃, CH₂Cl₂, CCl₃F, C₂Cl₆, C₂Cl₄ et C₂H₃Cl₃, les exemples portent uniquement sur la fluoration de produits saturés perhalogénés (CCl₄ et C₂Cl₃F₃). Or il est connu que la réactivité des molécules perhalogénées est très différente de celle des produits hydrogénés.

Ces derniers, tels que le F133a (1-chloro-2,2,2-trifluoroéthane), sont sensibles aux réactions d'élimination (perte d'HCI ou d'HF) et aux réactions de chloration qui conduisent à la formation de sous-produits non valorisables. Comme le montre le brevet FR 2 433 500, l'introduction d'oxygène à la température de la réaction (généralement plus élevée que celle utilisée pour fluorer les molécules perhalogénées) peut alors conduire à une baisse de la sélectivité.

L'oxyde de chrome, bien connu comme catalyseur de fluoration, est également un bon catalyseur d'oxydation de l'HCl (brevets US 4 803 065 et US 4 822 589). L'oxygène introduit au cours de la réaction de fluoration réagit sur l'HCl formé pour produire du chlore par la réaction de DEACON. Ce chlore peut alors facilement provoquer une chloration des produits hydrogénés présents dans le mélange réactionnel. Dans le cas de la fluoration du F133a en présence d'oxygène, il se forme ainsi principalement des produits de la série F120 (C₂HClₙF₅₋ₙ). Outre la formation de chlore, cette réaction de DEACON génère également de l'eau qui, pour des problèmes de corrosion est particulièrement indésirable dans un procédé de fluoration.

Pour pallier cet inconvénient, il a été proposé d'utiliser certains catalyseurs mixtes à base de chrome permettant de limiter la réaction de DEACON. Ainsi, le brevet EP 546 883 montre que, dans le cas de catalyseurs massiques, l'ajout d'un métal tel que le nickel permet d'inhiber partiellement l'oxydation de l'HCl. Un phénomène analogue est observé sur les catalyseurs mixtes Ni-Cr/AlF₃ (brevets EP 486 333 et WO 93/25507).

Dans une optique analogue, le brevet EP 328 127 propose de réaliser la réaction de fluoration du F133a en F134a sur un catalyseur ne contenant pas de chrome. Les solides préconisés contiennent au moins un métal choisi parmi le cobalt, le manganèse, le nickel, le palladium, l'argent, le ruthénium ou l'aluminium.

Récemment, après avoir montré que dans le cas de la réaction de fluoration du chlorure de méthylène en présence d'oxygène, les catalyseurs au chrome étaient peu sélectifs (formation de F22 et de dérivés halogénés de l'éthane), le brevet JP 5-339179 a également revendiqué l'utilisation de catalyseurs dépourvus de chrome, spécifiques à la synthèse du F32. Ces catalyseurs, tels que CoCl₂/AlF₃ ou NiCl₂/AlF₃, sont très sélectifs et leur stabilité est accrue par des additifs choisis parmi les terres rares (La, Ce) ou des éléments alcalino-terreux (Mg, Ca, Sr). Les durées de vie, réalisées en présence d'oxygène, sont importantes (150 jours), mais les productivités en F32 sont très faibles (< 10 g/h/l) et ne sont pas compatibles avec une production industrielle de F32.

Lors d'essais de fluoration du chlorure de méthylène, à temps de contact plus court, visant à augmenter la productivité en F32, nous avons été surpris de constater que, contrairement à ce que laissaient prévoir les brevets cités précédemment, des catalyseurs usuels de fluoration tels que Ni/AlF₃ ou Ni-Cr/AlF₃ ne sont pas stables, même en présence d'oxygène.

Par contre, il a maintenant été trouvé qu'il existe un domaine de température dans lequel un catalyseur à base de chrome pur (sans addition d'un autre oxyde métallique) peut produire en présence d'oxygène, avec une excellente stabilité, du F32 par fluoration en phase gazeuse du chlorure de méthylène, sans perte significative de sélectivité.

En effet, sans que l'on puisse l'expliquer, il a tout d'abord été surprenant de constater que la réaction de DEACON est pratiquement inexistante, même lorsque la réaction de fluoration du chlorure de méthylène est réalisée en présence de quantités importantes d'oxygène (3 % molaire), entre 250 et 450°C sur de l'oxyde de chrome. Les sous-produits provenant de réactions de chloration sont très minoritaires. De plus, l'absence de réaction de DEACON permet de limiter la génération d'eau dans le réacteur, ce qui limite les phénomènes de corrosion. La fluoration du chlorure de méthylène est donc une réaction très particulière, différente des réactions de fluoration telles que celles de F133a, C₂Cl₄, F123 ou F124.

Contrairement à ce que laissait prévoir l'art antérieur, il est donc possible d'utiliser un catalyseur à base de chrome pour réaliser cette réaction de fluoration en présence d'oxygène, sans diminution de la sélectivité de la réaction. Il n'est donc pas nécessaire d'utiliser des additifs particuliers pour en augmenter la sélectivité ; la suppression des additifs utilisés dans les catalyseurs mixtes permet de simplifier la fabrication du catalyseur et d'en diminuer par là même le coût.

L'utilisation d'un catalyseur à base de chrome (massique ou supporté) permet en outre d'atteindre des productivités en F32 très élevées. D'autre part, il a été surprenant de constater que, parmi les catalyseurs de fluoration testés, seuls les catalyseurs (massiques ou supportés), dont la phase active ne contient que du chrome sont capables de limiter la formation de coke à la température de fluoration du chlorure de méthylène.

Il a également été constaté que seul un domaine étroit de température permet de maintenir efficacement l'activité catalytique. En dessous de 330°C, l'apport d'oxygène ne permet pas de ralentir la formation du coke et le catalyseur se désactive progressivement. A l'opposé, une température supérieure à 400°C peut provoquer une cristallisation du solide, entraînant une diminution de son activité.

En résumé, il a été trouvé que, pour préparer du F32 avec une forte productivité, de manière stable et sélective, il faut combiner un apport d'oxygène, un catalyseur à base de chrome massique ou supporté, et un domaine de température restreint.

L'invention a donc pour objet un procédé de fabrication du F32 par fluoration catalytique en phase gazeuse du chlorure de méthylène (F30) au moyen d'acide fluorhydrique anhydre, caractérisé en ce que l'on opère en présence de 0,1 à 5 moles d'oxygène pour 100 moles de F30, à une température comprise entre 330 et 450°C, et avec un catalyseur au chrome, massique ou supporté.

Le précurseur utilisé pour préparer le catalyseur au chrome selon l'invention est préférentiellement un oxyde, un hydroxyde, un halogénure, un acétate ou un nitrate de chrome. Dans le cas d'un catalyseur massique, on choisit préférentiellement un solide à base de chrome de grande surface, partiellement fluoré, pouvant contenir éventuellement des éléments inertes tels que de l'alumine ou du graphite pour en accroître sa stabilité thermique et sa solidité. Le catalyseur peut également être obtenu par dépôt d'un dérivé du chrome sur un support inerte tel que de l'alumine ou de l'alumine partiellement fluorée. La teneur massique en chrome déposé sera alors préférentiellement inférieure à 20 %.

L'oxygène peut être introduit pur ou dilué dans un gaz inerte tel que l'azote. On utilise préférentiellement un rapport molaire O₂/CH₂Cl₂ compris entre 0,5 et 3 %.

Le rapport molaire HF/CH₂Cl₂ peut varier dans de larges limites. Il est généralement compris entre 1,5 et 10, de préférence entre 2 et 5.

Comme indiqué précédemment, la réaction doit être réalisée à une température comprise entre 330°C et 450°C. Cependant, il est préférable de travailler à une température comprise entre 350 et 400°C pour atteindre une productivité importante sans risquer une désactivation du catalyseur par cristallisation.

Le temps de contact, défini comme le rapport entre le débit total des réactifs (mesuré dans les conditions de réaction) et le volume de catalyseur, peut varier dans de larges limites et est généralement compris entre 0,01 et 10 secondes. En pratique, on préfère travailler à des temps de contact compris entre 0,05 et 5 secondes.

La réaction peut être réalisée à pression atmosphérique ou à une pression plus élevée. On choisit préférentiellement une pression comprise entre 1 et 20 bars absolus.

Les exemples suivants illustrent l'invention sans la limiter.

### PREPARATION ET ACTIVATION DES CATALYSEURS

### • Catalyseur massique (A)

Un oxyde de chrome massique ayant une surface spécifique de 209 m²/g et un volume poreux (4 nm < r < 63 µm) de 0,1 ml est utilisé après une activation par l'HF anhydre.

Pour cela, l'oxyde de chrome est tout d'abord séché à 200°C puis traité par un mélange N₂/HF à 200°C. Lorsque l'exothermie initiale est passée, on augmente la température jusqu'à 380°C. Le catalyseur est ensuite maintenu à 380°C, sous un courant d'HF anhydre pur, pendant 18 heures.

Le catalyseur (A) activé a les propriétés physico-chimiques suivantes :

| | |
|---|---|
| Teneur pondérale en fluor | 27 % |
| Teneur pondérale en chrome | 53 % |
| Volume des pores d'un rayon compris entre 4 nm et 63 µm | 0,13 ml/g |
| Surface BET | 101 m²/g |

### • Catalyseurs supportés (B), (C) et (D)

Dans un évaporateur rotatif, on place 250 ml d'alumine partiellement fluorée (contenant globalement 83 % en masse de fluorure d'aluminium et 16 % d'alumine), préalablement obtenue par fluoration d'alumine vers 300°C à l'aide d'azote et d'acide fluorhydrique. Ce support fluoré présente avant imprégnation les caractéristiques physico-chimiques suivantes :

| | |
|---|---|
| forme | billes de 1-2 mm de diamètre |
| densité apparente | 0,57 g/ml |
| surface BET | 67 m²/g |
| volume poreux | 0,72 ml/g (pour les pores de rayon compris entre 4 nm et 63 µm) |

En utilisant les quantités indiquées dans le tableau I, on prépare par ailleurs une solution aqueuse (Solution 1) contenant les précurseurs des métaux souhaités. Dans le cas des catalyseurs (B) et (D) préparés à partir de CrO₃, l'imprégnation est réalisée en milieu méthanolique, de manière à réduire le chrome au degré d'oxydation III. Pour cela, on ajoute simultanément, sur le support en agitation, la solution aqueuse contenant le chrome et une solution méthanolique (Solution 2).

**TABLEAU I**

| | **Solution 1** | **Solution 2** |
|---|---|---|
| Catalyseur B | CrO₃ = 32,5 g H₂O = 70g | CH₃OH = 46 g H₂O = 15 g |
| Catalyseur C | 20g NiCl₂ 6 H₂O H₂O = 95 g | néant |
| Catalyseur D | CrO₃ = 12,5 g NiCl₂ . 6 H₂O = 29 g H₂O = 40 g | CH₃OH = 17,8 g H₂O = 50g |

L'imprégnation est réalisée en 45 minutes, à température ambiante et sous pression atmosphérique, sur le support en agitation. Le catalyseur est ensuite séché sous courant d'azote, en lit fluidisé, vers 110°C pendant 4 heures.

Le catalyseur est ensuite chargé dans un réacteur en Inconel 600 et activé en lit fixe par un mélange azote/HF suivant la procédure décrite dans le brevet EP 0 486 333. Le Tableau II suivant indique la composition chimique des catalyseurs ainsi activés.

**TABLEAU II**

| **Catalyseur** | **Composition Chimique (pondérale)** |
|---|---|
| **B** | Cr = 12% ; F = 56 % ; Al = 25 % |
| **C** | Ni = 3 % ; F = 66 % ; Al = 30 % |
| **D** | Cr = 6 % ; Ni = 6% ; F = 57 % : Al = 26 % |

### FLUORATION DU CHLORURE DE METHYLENE

### EXEMPLE 1

Dans un réacteur tubulaire en Inconel 600 de 1 cm de diamètre intérieur et d'un volume de 40 ml, on charge 4 ml d'oxyde de chrome préalablement fluoré (catalyseur A). On introduit dans un premier temps l'HF et l'air avec des débits respectifs de 0,68 mole/h et 0,03 mole/h. Le chlorure de méthylène, vaporisé dans un préchauffeur, dont la température est fixée à 150°C, est ensuite introduit sous forme gazeuse dans le réacteur à un débit de 0,23 mole/h. La réaction est réalisée à pression atmosphérique. La température du réacteur est maintenue à 350°C et le temps de contact dans ces conditions est de 0,3 seconde.

Les produits de réaction sont ensuite lavés, séchés et analysés par chromatographie en phase gazeuse. Les résultats sont regroupés dans le Tableau III suivant.

**TABLEAU III**

| **Temps (h)** | **Conversion F30 (% molaire)** | **Sélectivité F31 (% molaire)** | **Sélectivité F32 (% molaire)** |
|---|---|---|---|
| 24 | 64 | 27 | 73 |
| 151 | 60 | 28 | 72 |
| 201 | 62 | 27 | 73 |
| 314 | 63 | 27 | 73 |
| 428 | 61 | 27 | 73 |
| 524 | 63 | 27 | 73 |
| 640 | 62 | 28 | 72 |
| 893 | 60 | 27 | 73 |
| 971 | 61 | 27 | 73 |

La mesure du rapport O₂/N₂ permet par ailleurs de vérifier si l'oxygène introduit sous forme d'air a été consommé. Dans ces conditions de réaction, 5 % de l'oxygène introduit est converti en CO; la formation de CO₂ est marginale. Les autres sous-produits (F23 et F40) sont en quantités inférieures à 700 ppm. Le rapport molaire O₂/N₂ en sortie de réacteur est de 0,26 ce qui indique l'absence de réaction de DEACON.

On constate que ces conditions de réaction permettent de maintenir une activité parfaitement stable avec une très forte productivité en F32 (1350 g/h/l) et une sélectivité en F31+F32 supérieure à 99,7 %.

### EXEMPLE COMPARATIF 1

La réaction est réalisée dans les mêmes conditions qu'à l'exemple 1 mais à une température de 300°C. Le débit des réactifs est ajusté de manière à maintenir un temps de contact de 0,3 seconde. Les résultats sont regroupés dans le tableau suivant.

**TABLEAU IV**

| **Temps (h)** | **Conversion F30 (% molaire)** | **Sélectivité F31 (% molaire)** | **Sélectivité F32 (% molaire)** |
|---|---|---|---|
| 24 | 54 | 24 | 76 |
| 96 | 42 | 27 | 73 |
| 220 | 40 | 31 | 69 |
| 321 | 33 | 34 | 66 |

L'oxygène introduit ne réagit pratiquement pas ; la quantité de CO/CO₂ formé est inférieure au seuil de détection (< 0,05 %).

Le catalyseur s'est désactivé par cokage et contient après 321 heures de marche 2,5 % (massique) de carbone.

On constate qu'une température de 300°C ne permet pas de maintenir une activité stable sur ce catalyseur. Cette température permet d'atteindre une productivité en F32 élevée (1200 g/h/l) mais est insuffisante pour supprimer en continu la formation du coke ou de ces précurseurs.

### EXEMPLE 2

La réaction de fluoration du chlorure de méthylène est réalisée dans les mêmes conditions que dans l'exemple 1 sur le catalyseur supporté B (Cr/AlF₃). Le tableau V regroupe les résultats obtenus.

**TABLEAU V**

| **Temps (h)** | **Conversion F30 (% molaire)** | **Sélectivité F31 (% molaire)** | **Sélectivité F32 (%molaire)** |
|---|---|---|---|
| 90 | 57,6 | 29,4 | 70,6 |
| 226 | 55,7 | 30,5 | 69,4 |
| 274 | 53,7 | 32,9 | 67,0 |
| 372 | 55,9 | 30,8 | 69,1 |
| 493 | 54,9 | 31,6 | 68,2 |
| 564 | 53,7 | 32,1 | 67,8 |

L'oxygène introduit réagit très peu ; la quantité de CO/CO₂ formé est inférieure au seuil de détection (< 0.05 %). On note la formation de 100 ppm de bis(fluorométhyl)éther.

### EXEMPLES COMPARATIFS 2 ET 3

La réaction est réalisée dans les conditions de l'exemple 1 sur les catalyseurs supportés C et D (Ni/AlF₃ et Ni-Cr/AlF₃) non conformes à la présente invention.

La conversion du chlorure de méthylène et les sélectivités en F32 et F31 sont indiquées dans le tableau VI suivant :

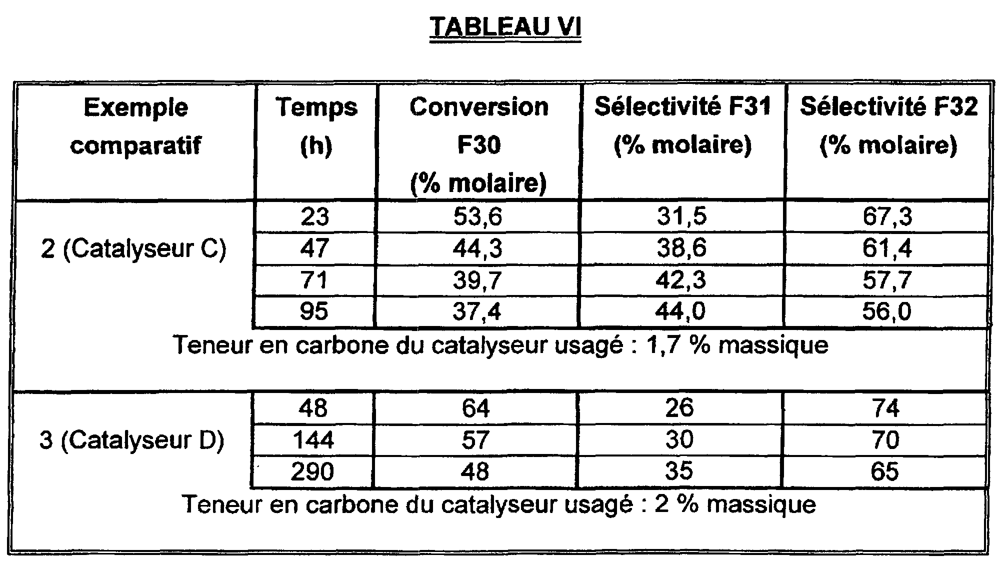

On constate que les catalyseurs de fluoration C et D ne permettent pas d'atteindre la durée de vie obtenue sur les catalyseurs A et B à base de chrome seul (massique ou supporté) et se désactivent par cokage malgré l'apport continu d'air et la température élevée.

Sur ces catalyseurs, il se forme moins de 500 ppm de CO/CO₂ et il ne se forme pas de bis(fluorométhyl)éther (< 10 ppm). L'oxygène introduit ne réagit pas.

Ces exemples comparatifs 2 et 3 montrent qu'il est nécessaire d'avoir un catalyseur contenant uniquement du chrome (catalyseurs A et B), pour que l'air introduit puisse supprimer ou inhiber la formation du coke à la température de fluoration du chlorure de méthylène.

## Revendications

1. Procédé de fabrication du difluorométhane par fluoration catalytique en phase gazeuse du chlorure de méthylène (CH₂Cl₂) au moyen d'acide fluorhydrique anhydre (HF), **caractérisé en ce que** l'on opère en présence de 0,1 à 5 moles d'oxygène pour 100 moles de chlorure de méthylène, à une température comprise entre 330 et 450°C, avec un catalyseur massique ou supporté, dont la phase active ne contient que du chrome, et avec un rapport molaire HF/ CH₂Cl₂ compris entre 1,5 et 10.

2. Procédé selon la revendication 1, dans lequel le rapport molaire HF/ CH₂Cl₂ est compris entre 2 et 5.

3. Procédé selon la revendication 1 ou 2, dans lequel le rapport molaire O₂/CH₂Cl₂ est compris entre 0,5 % et 3 %.

4. Procédé selon l'une des revendications 1 à 3, dans lequel on opère à une température comprise entre 350 et 400°C.

5. Procédé selon l'une des revendications 1 à 4, dans lequel on utilise un catalyseur au chrome massique.

6. Procédé selon l'une des revendications 1 à 4, dans lequel on utilise un catalyseur au chrome supporté, la teneur pondérale en chrome étant de préférence inférieure à 20 %.

7. Procédé selon l'une des revendications 1 à 6, dans lequel le temps de contact est compris entre 0,01 et 10 secondes, de préférence entre 0,05 et 5 secondes.

8. Procédé selon l'une des revendications 1 à 7, dans lequel on opère à une pression comprise entre 1 et 20 bars absolus.

## Claims

1. Process for the manufacture of difluoromethane by gas-phase catalytic fluorination of methylene chloride (CH₂Cl₂) by means of anhydrous hydrofluoric acid (HF), **characterized in that** the operation is carried out in the presence of 0.1 to 5 mol of oxygen per 100 mol of methylene chloride, at a temperature of between 330 and 450°C, with a bulk or supported catalyst, the active phase of which contains only chromium, and with an HF/CH₂Cl₂ molar ratio of between 1.5 and 10.

2. Process according to Claim 1, in which the HF/CH₂Cl₂ molar ratio is between 2 and 5.

3. Process according to Claim 1 or 2, in which the O₂/CH₂Cl₂ molar ratio is between 0.5% and 3%.

4. Process according to one of Claims 1 to 3, in which the operation is carried out at a temperature of between 350 and 400°C.

5. Process according to one of Claims 1 to 4, in which a bulk chromium catalyst is employed.

6. Process according to one of Claims 1 to 4, in which a supported chromium catalyst is employed, the weight content of chromium being preferably lower than 20%.

7. Process according to one of Claims 1 to 6, in which the contact time is between 0.01 and 10 seconds, preferably between 0.05 and 5 seconds.

8. Process according to one of Claims 1 to 7, in which the operation is carried out at a pressure of between 1 and 20 bar absolute.

## Patentansprüche

1. Verfahren zur Herstellung von Difluormethan durch katalytische Gasphasenfluorierung von Methylenchlorid (CH₂Cl₂) mittels Fluorwasserstoff, **dadurch gekennzeichnet, daß** man in Gegenwart von 0,1 bis 5 mol Sauerstoff auf 100 mol Methylenchlorid bei einer Temperatur zwischen 330 und 450 °C mit einem Katalysator in Masse oder einem Trägerkatalysator, dessen aktive Phase nur Chrom enthält, und mit einem HF/CH₂Cl₂-Molverhältnis zwischen 1,5 und 10 verfährt.

2. Verfahren nach Anspruch 1, wobei das HF/CH₂Cl₂-Molverhältnis zwischen 2 und 5 liegt.

3. Verfahren nach Anspruch 1 oder 2, wobei das O₂/CH₂Cl₂-Verhältnis zwischen 0,5 % und 3 % liegt.

4. Verfahren nach einem der Ansprüche 1 bis 3, wobei man bei einer Temperatur zwischen 350 und 400 °C verfährt.

5. Verfahren nach einem der Ansprüche 1 bis 4, wobei man einen Chromkatalysator in Masse verwendet.

6. Verfahren nach einem der Ansprüche 1 bis 4, wobei man einen Chromträgerkatalysator verwendet, wobei der Gewichtsgehalt an Chrom vorzugsweise kleiner als 20 % ist.

7. Verfahren nach einem der Ansprüche 1 bis 6, wobei die Kontaktzeit zwischen 0,01 und 10 Sekunden, vorzugsweise zwischen 0,05 und 5 Sekunden, liegt.

8. Verfahren nach einem der Ansprüche 1 bis 7, bei dem man bei einem Druck zwischen 1 und 20 bar absolut verfährt.
